# EUROPEAN PATENT APPLICATION

(11) **EP 4 699 629 A1**
(43) Date of publication of application: **25.02.2026**
(21) Application number: 24807423.9
(22) Date of filing: 08.05.2024
(51) Int. Cl.: A61M 5/142, A61M 5/168

(54) **DRUG INJECTION DEVICE**

(30) Priority: 16.05.2023 KR 20230062982
(71) Applicant: Caremedi Co., Ltd., Seoul 07207 (KR); Sogang University Research & Business Development Foundation, Seoul 04107 (KR)
(72) Inventor: SHIN, Woonsup, Seoul 04065 (KR); ZHU, Enhua, Seoul 04109 (KR)
(74) Representative: Manitz Finsterwald Patent- und Rechtsanwaltspartnerschaft mbB
(86) International application number: PCT/KR2024/006149
(87) International publication number: WO 2024/237547

(57) **Abstract**

A patch-type drug injection pump according to an embodiment of the present disclosure includes: a housing including a lid and a contact surface; and components disposed in a mounting space between the lid and the contact surface. The components include: a drug reservoir in which a drug to be injected is stored; a pump assembly configured to be driven to transfer the drug in the drug reservoir toward an injection target; a circuit unit equipped with electronic components necessary for driving and controlling the pump; and an injection assembly equipped with an insertion tube to be inserted into the injection target. The injection assembly is disposed at a central region of the mounting space. Further, the drug reservoir, the pump assembly, and the circuit unit are disposed to surround the injection assembly.

## Description

### TECHNICAL FIELD

The technical field of the present disclosure relates to a patch-type drug injection device.

### BACKGROUND

Drugs may be injected into the body in various ways, such as oral, subcutaneous, and intravenous administration, depending on the type of drug and the purpose and method of treatment. A drug injector using a drug pump may automatically inject a drug into the body at a desired speed and dose at a required time. Therefore, a drug injector using a drug pump may be used not only in hospitals or in patients' daily living environments, but also in various forms.

An insulin pump, which is commonly called an insulin injector, is a medical device that supplies insulin into the body from the outside at a predetermined time to accurately control the blood sugar level, similar to the pancreas, for a diabetic patient who does not secrete insulin or secretes only a small amount of insulin.

Such an insulin pump is used by insulin-dependent diabetic patients, and may continuously inject a drug for 24 hours in a state of being attached to the patient. In this way, the insulin injector needs to periodically inject a drug into a diabetic patient for a long period of time. Therefore, technological development is actively underway to miniaturize and automate insulin injectors for the convenience of users.

In general, an insulin injector is attached to a user's body for a predetermined period of use. However, an insertion tube that has been inserted into the user may become detached due to the user's movement or an external impact.

Accordingly, there is a need for a device capable of maintaining the inserted tube even in the presence of the user's movement or external impact, thereby enabling a stable injection of an accurate amount of drug into the patient.

### DISCLOSURE OF THE INVENTION

### PROBLEMS TO BE SOLVED BY THE INVENTION

In view of the foregoing, the present disclosure is conceived to provide a drug injection device in which an injection assembly configured to inject a drug is disposed at a central portion.

The problems to be solved by the present disclosure are not limited to the above-described problems. There may be other problems to be solved by the present disclosure.

### MEANS FOR SOLVING THE PROBLEMS

As a means for solving the above-described problems, a drug injection device according to an embodiment of the present disclosure includes: a housing including a lid and a contact surface; and components disposed in a mounting space between the lid and the contact surface. The components include: a drug reservoir in which a drug to be injected is stored; a pump assembly configured to be driven to transfer the drug in the drug reservoir toward an injection target; a circuit unit equipped with electronic components necessary for driving and controlling the pump; and an injection assembly equipped with an insertion tube to be inserted into the injection target. The injection assembly is disposed at a central region of the mounting space. Further, the drug reservoir, the pump assembly, and the circuit unit are disposed to surround the injection assembly.

### EFFECTS OF THE INVENTION

According to the means for solving the problems of the present disclosure, an injection assembly is disposed at a central region. Thus, an insertion tube remains stably inserted into a user even when a housing is displaced by the user's movement or an external impact.

### BRIEF DESCRIPTION OF THE DRAWINGS

**FIG. 1** is a front perspective view of a drug injection device according to an embodiment of the present disclosure.
**FIG. 2** is a rear perspective view of the drug injection device according to an embodiment of the present disclosure.
**FIG. 3** is an exploded perspective view of a housing illustrated in **FIG. 1****.**
**FIG. 4** is an internal plan view of the housing illustrated in **FIG. 1****.**
**FIG. 5** is a plan view of a drug reservoir illustrated in **FIG. 4****.**
**FIG. 6** is a perspective view of a pump assembly illustrated in **FIG. 4****.**
**FIG. 7** is a cross-sectional view of a port connector illustrated in **FIG. 6****.**
**FIG. 8** is a block diagram schematically illustrating a configuration of an electroosmotic pump illustrated in **FIG. 6****.**
**FIG. 9** is a block diagram schematically illustrating a configuration of a driver illustrated in **FIG. 8****.**
**FIG. 10** is an example diagram schematically illustrating an operation of the driver illustrated in **FIG. 9****.**
**FIG. 11** is a perspective view of an injection assembly illustrated in **FIG. 4****.**
**FIG. 12** is an enlarged view of a portion A illustrated in **FIG. 3****.**
**FIG. 13** is a perspective view illustrating the injection assembly coupled to the configuration shown in **FIG. 12****.**
**FIG. 14** and **FIG. 15** are rear perspective views of the drug injection device according to an embodiment of the present disclosure.

### BEST MODE FOR CARRYING OUT THE INVENTION

Hereafter, embodiments will be described in detail with reference to the accompanying drawings. However, it is to be noted that the present disclosure is not limited to the embodiments but can be embodied in various other ways. Also, the accompanying drawings are provided only for a better understanding of the embodiments disclosed in the present disclosure and are not intended to limit technical ideas disclosed in the present disclosure. In the drawings, parts irrelevant to the description are omitted for the simplicity of explanation, and the size, form and shape of each component illustrated in the drawings can be modified in various ways. Like reference numerals denote like parts throughout the whole document.

Suffixes "module" and "unit" used for components disclosed in the following description are merely intended for easy description of the specification, and the suffixes themselves do not give any special meaning or function. Further, in the following description of the present disclosure, a detailed explanation of known related technologies may be omitted to avoid unnecessarily obscuring the subject matter of the present disclosure.

Throughout the whole document, the term "connected to (contacted with or coupled to)" may be used to designate a connection or coupling of one element to another element and includes both an element being "directly connected to (contacted with or coupled to)" another element and an element being "indirectly connected to (contacted with or coupled to)" another element via another element. Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

Further, in describing components of the present disclosure, ordinal numbers such as first, second, etc. can be used only to differentiate the components from each other, but do not limit the sequence or relationship of the components. For example, a first component of the present disclosure may also be referred to as a second component and vice versa.

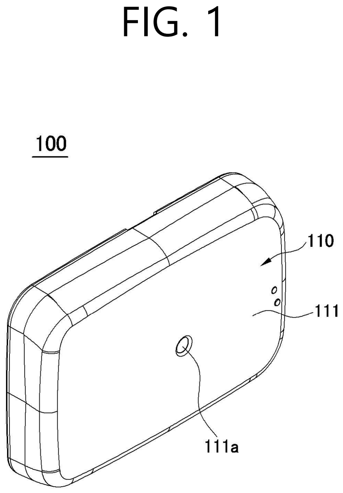

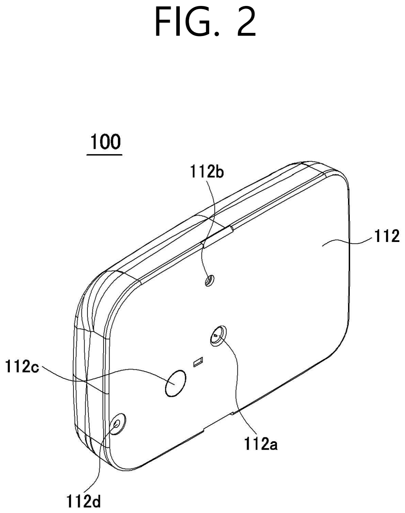

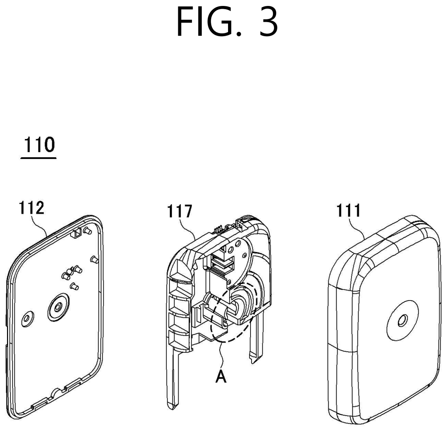

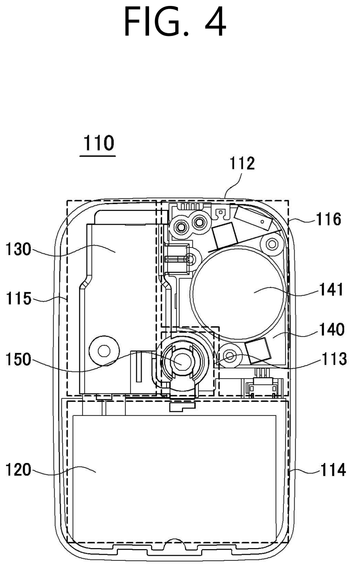

**FIG. 1** is a front perspective view of a drug injection device according to an embodiment of the present disclosure, **FIG. 2** is a rear perspective view of the drug injection device according to an embodiment of the present disclosure, **FIG. 3** is an exploded perspective view of a housing illustrated in **FIG. 1****,** and **FIG. 4** is an internal plan view of the housing illustrated in **FIG. 1****.**

Referring to **FIG. 1** to **FIG. 4****,** a patch-type drug injection device 100 according to an embodiment of the present disclosure includes a housing 110 and components disposed within the housing 110.

The housing 110 includes a lid 111 and a contact surface 112, and a mounting space is formed between the lid 111 and the contact surface 112. The lid 111 has rounded corners, and, thus, the patch-type drug injection device 100 can remain continuously attached to an injection target without a foreign body sensation. The contact surface 112 is configured to be attached to the injection target, and a discharge port 112a is formed therein to draw an insertion tube outward. Herein, a silicone cap may be coupled to the discharge port 112a, thereby fixing the insertion tube that has been inserted into the injection target.

The mounting space is divided into a central region 113 and first to third regions 114, 115 and 116. The first to third regions 114, 115 and 116 are formed to surround the central region 113. In particular, the mounting space within the housing 110 is divided into the central region 113 and the first to third regions 114, 115 and 116, which extend in directions perpendicular to an insertion direction of the insertion tube. Major components are coupled to each of the regions. In a conventional device, a PCB circuit unit is first disposed over the entire or most of the plane of the device, and a drug reservoir or pump is disposed above the circuit unit, which causes the overall thickness of the device to increase. However, in the patch-type drug injection device 100 according to the present disclosure, the thickness of the device 100 is reduced by minimizing the overlapping areas between components.

The housing 110 may further include a case frame 117 configured to fix the components disposed in the mounting space. The case frame 117 may be configured to define the central region 113 and the first to third regions 114, 115 and 116. Further, the case frame 117 is coupled between the lid 111 and the contact surface 112.

The components include the drug reservoir 120, the pump assembly 130, the circuit unit 140, and the injection assembly 150, and may be disposed in the central region 113 and the first to third regions 114, 115 and 116 described above. For example, the drug reservoir 120 may be disposed in the first region 114, the pump assembly 130 may be disposed in the second region 115, the circuit unit 140 may be disposed in the third region 116, and the injection assembly 150 is disposed in the central region 113. The drug reservoir 120, the pump assembly 130, and the circuit unit 140 may be disposed to surround the injection assembly 150.

Accordingly, since the injection assembly 150 including the insertion tube is disposed in the central region 113, the patch-type drug injection device 100 can remain attached to the user without the insertion tube being detached, even when shaking occurs due to the user's movement.

Hereafter, the drug reservoir 120, the pump assembly 130, the circuit unit 140, and the injection assembly 150 will be described in detail.

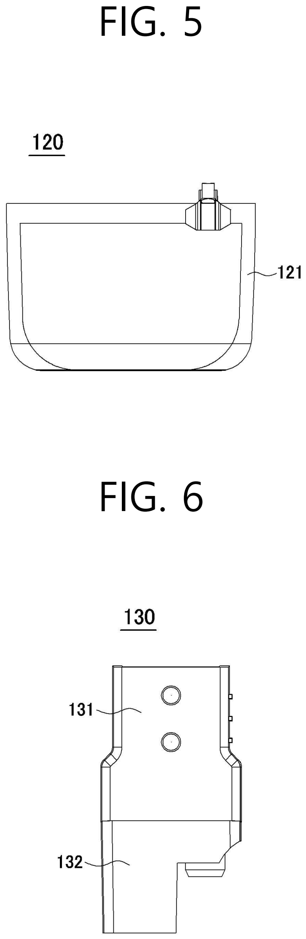

**FIG. 5** is a plan view of the drug reservoir illustrated in **FIG.** 4. Referring to **FIG. 5****,** the drug reservoir 120 stores a drug to be injected, and its thickness varies depending on the amount of drug contained therein. The drug reservoir 120 may be provided as a flat pouch-type bag formed of a polymer material. A sealing portion 121 may be formed along the periphery of the drug reservoir 120 by bonding the edges of its two surfaces over a predetermined width. The sealing portion 121 may restrict expansion of the drug reservoir 120 toward its periphery.

The drug reservoir 120 may be formed of a polymer film or the like, and inner and outer layers of the drug reservoir 120 may be formed of materials having different melting points. Herein, the melting point of the inner layer is lower than that of the outer layer. The drug reservoir 120 is manufactured by applying heat at a predetermined temperature sufficient to melt the inner material to form the sealing portion 121. When heat is applied to a side surface of the sealing portion 121, the inner materials adhere to each other to form a seal. Also, when a lower portion is folded into a W-shape and heated, the inner materials adhere to each other, whereas the outer materials do not, which results in a corrugated seal. Unlike conventional techniques, the drug reservoir 120 is thinly formed of a flexible material. Thus, the patch-type drug injection device 100 can be implemented to be thinner and lighter.

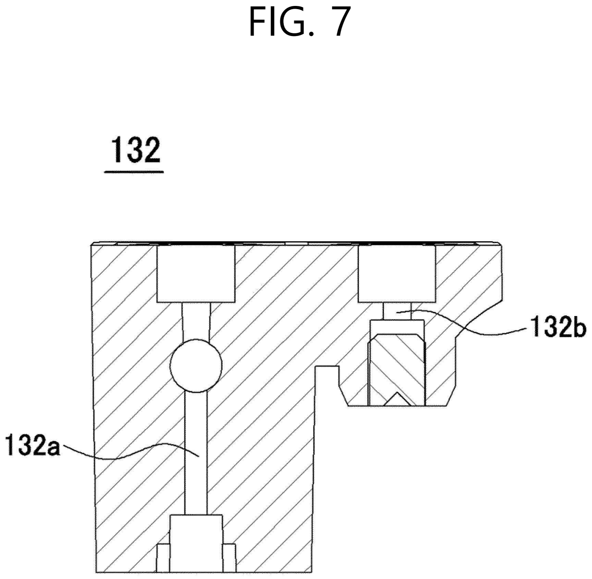

**FIG. 6** is a perspective view of a pump assembly illustrated in **FIG. 4****,** and **FIG. 7** is a cross-sectional view of a port connector illustrated in **FIG. 6****.**

Referring to **FIG. 6****,** the pump assembly 130 is configured to suck a drug from the drug reservoir 120 and deliver it toward the injection assembly 150. The pump assembly 130 includes an electroosmotic pump 131 and a port connector 132. The electroosmotic pump 131 is an electrochemically driven pump, and will be described in detail later. Referring to **FIG. 7****,** the port connector 132 includes an inflow path 132a through which the drug flows from the drug reservoir 120 to the electroosmotic pump 131 and a discharge path 132b through which the drug is discharged from the electroosmotic pump 131 to the injection assembly 150.

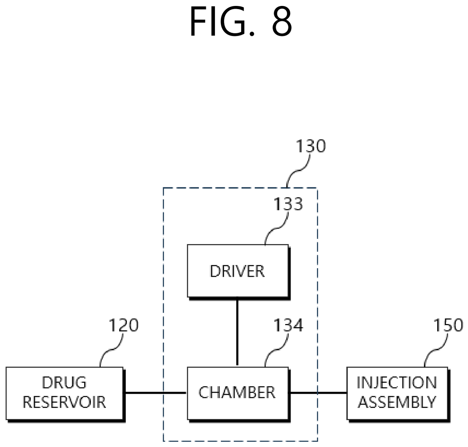

Hereafter, the electroosmotic pump 131 will be described in detail with reference to **FIG. 8** to **FIG. 10****.**

**FIG. 8** is a block diagram schematically illustrating a configuration of the electroosmotic pump 131 illustrated in **FIG. 6****.**

Referring to **FIG. 8****,** the electroosmotic pump 131 includes a driver 133 and a chamber 134. The driver 133 is electrochemically driven in response to a control signal to generate positive and negative pressures. Thus, a drug is sucked from the drug reservoir 120 and then stored in the chamber 134. The drug stored in the chamber 134 is discharged to the injection assembly 150. The chamber 134 receives and contains the drug sucked from the drug reservoir 120 according to a pressure generated by the driver 133. Herein, the drug needs to be injected into a specific patient and may be, for example, insulin that is injected into a diabetic patient.

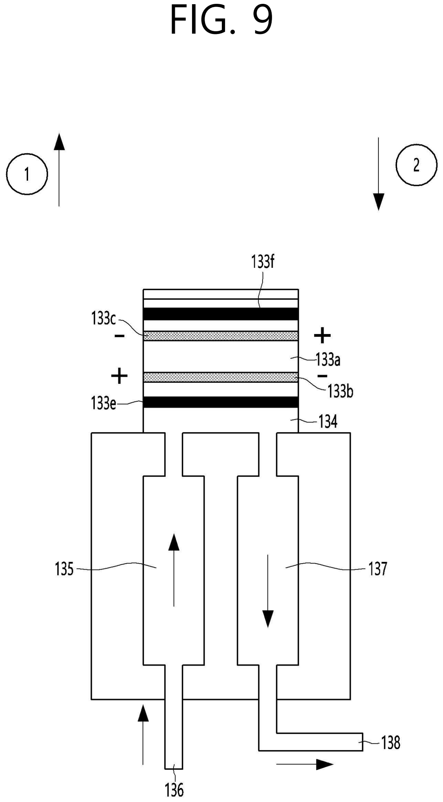

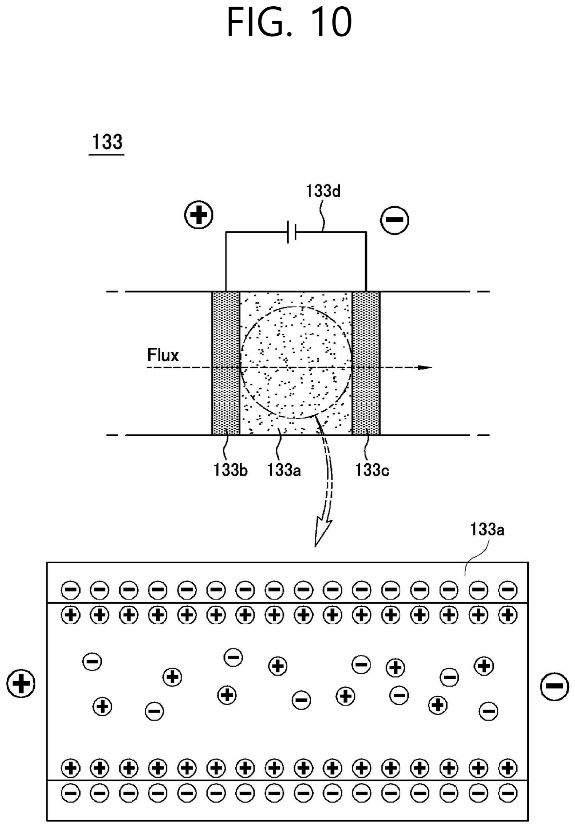

**FIG. 9** is a block diagram schematically illustrating a configuration of a driver illustrated in **FIG. 8****,** and **FIG. 10** is an example diagram schematically illustrating an operation of the driver illustrated in **FIG. 9****.**

The driver 133 will be described in detail with reference to **FIG. 7** and **FIG. 8****.** The driver 133 is a pump that uses the movement of a fluid according to electroosmosis that occurs when a voltage or current is applied by using electrodes at both ends of a pumping unit (membrane) 133a. The driver 133 may include the membrane 133a, a power source 133d that applies a voltage or current to a first electrode 133b and a second electrode 1331c arranged on both sides of the membrane 133a, and first and second diaphragms 133e and 133f through which a fluid moves. The first and second diaphragms 133e and 133f are respectively provided on one side and on the other side of the pumping unit 133a, and are deformed in shape by the movement of a pumping solution as positive and negative pressures are alternately generated. For example, the first diaphragm 133e and the second diaphragm 133f transfer the positive and negative pressures generated by an operation of the pumping unit 133a to a transfer target fluid. More specifically, when a negative pressure is generated, at least a part of the first diaphragm 133e and the second diaphragm 133f moves backwards (*i.e.,* moves in a direction ①) such that the transfer target fluid is sucked to the chamber 134, and when a positive pressure is generated, at least a part of the first diaphragm 133e and the second diaphragm 133f moves forwards (*i.e.,* moves in a direction ②) such that the transfer target fluid is discharged from the chamber 134.

Silica, glass, and the like are generally used as a material of the membrane 133a, and when the silica and glass are immersed in an aqueous solution, surfaces thereof are negatively charged. There are many paths, through which fluids may pass, in the membrane 133a, and when one of the paths is enlarged, a surface of the fluid path with negative charges (bound anions) may be in a state in which the negative charges are balanced by mobile cations having positive charges. In this state, when a positive voltage is applied to the first electrode 133b and a negative voltage is applied to the second electrode 133c, a negative pressure is generated, and the fluid inside the driver 133 moves in the direction ① due to the negative pressure. In this case, the drug is sucked through an inflow path 136 and flows into the chamber 134 through a suction valve 135. In this case, a discharge valve 137 is closed such that the negative pressure is not transferred to an outflow path 138. When a negative voltage is applied to the first electrode 133b and a positive voltage is applied to the second electrode 133c, a positive pressure in the opposite direction is generated by a reversible electrochemical reaction. The fluid inside the driver 133 moves in the direction ② by the positive pressure. In this case, the drug stored in chamber 134 is injected into a target through the discharge valve 137 and the outflow path 138. In this case, the suction valve 135 is closed such that the positive pressure is not transferred to the inflow path 136.

This phenomenon is called electroosmosis, and a pump that uses this principle is an electroosmotic pump 131. Since the patch-type drug injection device 100 of the present disclosure uses the electroosmotic pump 131 as a driving means, the driving means can be manufactured to be thinner than in other technologies that use an electric motor or the like. Accordingly, the overall thickness of the patch-type drug injection device 100 can be reduced, and the device 100 can be more stably attached to the skin.

Referring back to **FIG. 4****,** the circuit unit 140 is equipped with electronic components necessary for driving and controlling the pump assembly 130, and may include a battery 141 for supplying power. Also, the injection assembly 150 may include the insertion tube to inject a drug discharged from the pump assembly 130 into an injection target.

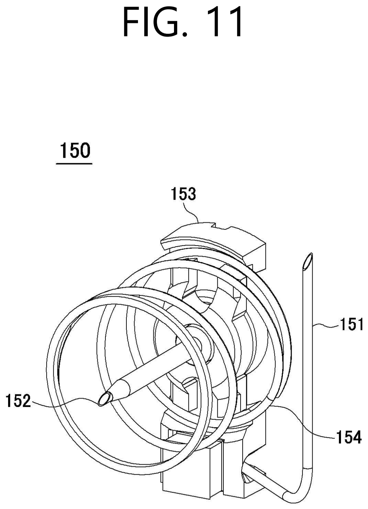

**FIG. 11** is a perspective view of the injection assembly illustrated in **FIG. 4****.** Referring to **FIG. 11****,** the insertion tube of the injection assembly 150 may be composed of only a needle 151, or may be composed of the needle 151 and a cannula 152.

**FIG. 11** illustrates an embodiment in which the insertion tube is composed of the needle 151 and the cannula 152. The injection assembly 150 may include a needle pusher 153, in which the needle 151 and the cannula 152 are coupled, and a return spring 154.

The needle 151 is coupled to the discharge path 132b of the port connector 132, and extends from the discharge path 132b toward the discharge port 112a of the contact surface 112. The cannula 152, into which the needle 151 is inserted, is disposed toward the discharge port 112a. The needle pusher 153, to which a part of the needle 151 is fixed, is configured to be moved by an external force to insert the needle 151 and the cannula 152 into the injection target. The return spring 154 contracts and relaxes along a moving direction of the needle pusher 153 to return the needle pusher 153 to its original position.

Moreover, the injection assembly 150 may further include a guide portion. The guide portion, into which a part of the needle pusher 153 is inserted, guides the movement of the needle pusher 153. Herein, the guide portion may be formed in the case frame 117.

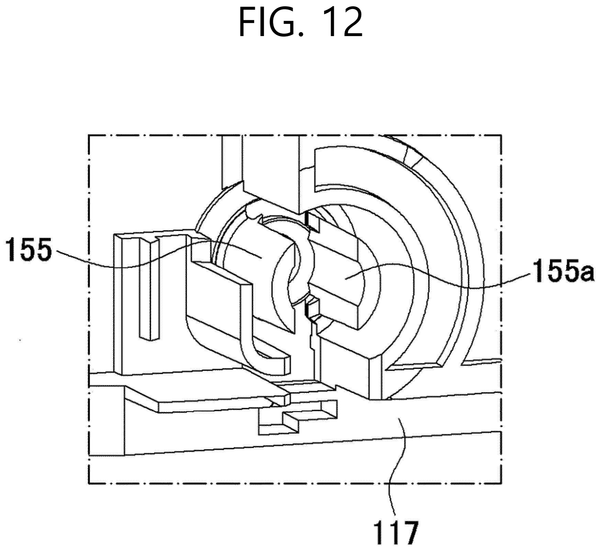

**FIG. 12** is an enlarged view of a portion A illustrated in **FIG. 3****.** Referring to **FIG. 12****,** a guide portion 155 is formed into a cylindrical shape extending along an insertion direction of the needle 151, and includes a guide groove 155a which extends in the insertion direction of the needle 151 and into which a part of the needle pusher 153 is inserted.

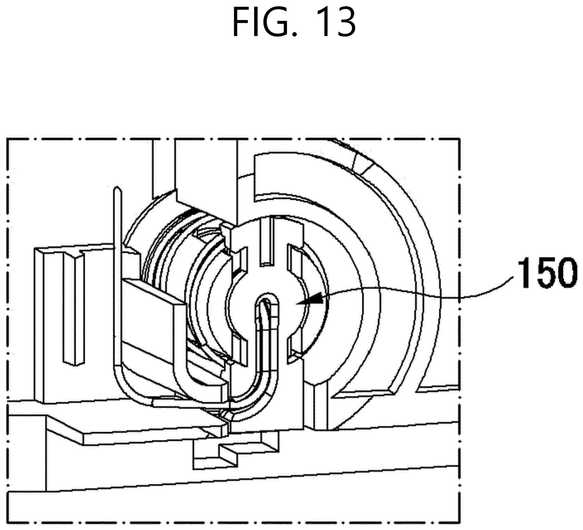

**FIG. 13** is a perspective view illustrating the injection assembly 150 coupled to the case frame 117. A part of the needle pusher 153 is inserted and coupled to the guide groove 155a of the guide portion 155, and the injection assembly 150 moves along the guide portion 155.

Hereafter, referring to **FIG. 1** and **FIG. 2****,** additional components of the patch-type drug injection device 100 will be described.

Referring to **FIG. 1****,** the lid 111 of the housing 110 has a shooting hole 111a formed in a region corresponding to the injection assembly 150. The shooting hole 111a is a hole into which a shooting member of a shooting device (not shown) is inserted to press the needle pusher 153, thereby allowing the needle 151 and the cannula 152 to be inserted into the injection target. After the shooting member passes through the shooting hole 111a, it collides with the needle 151 and the needle pusher 153. Then, by a force with which the shooting member pushes the needle 151 and the needle pusher 153, the needle 151 and the needle pusher 153 are moved toward the discharge port 112a.

Referring to **FIG. 2****,** a drug injection hole 112b and an air vent hole 112c are formed in the contact surface 112 of the housing 110. The drug injection hole 112b is a hole for supplying a drug from the outside into the drug reservoir 120, and the air vent hole 112c is a hole for reducing a pressure difference between the inside and the outside of the housing 110. The drug injection hole 112b is formed at a position corresponding to a drug injection path (not shown) formed in the port connector 132. Thus, a drug can be injected through the drug injection hole 112b and the drug injection path by means of a syringe or the like.

Further, the patch-type drug injection device 100 may include a sound output device (not shown) within the housing 110 to provide a notification of a specific condition. The circuit unit 140 includes a sound output circuit capable of stopping the operation of the sound output device (not shown), and a buzzer stop hole 112d may be formed in the contact surface 112 to provide a passage for interrupting the sound output circuit of the circuit unit 140. Herein, silicone caps may be coupled to the discharge port 112a and the drug injection hole 112b, and air vent stickers may be attached to the air vent hole 112c and the buzzer stop hole 112d.

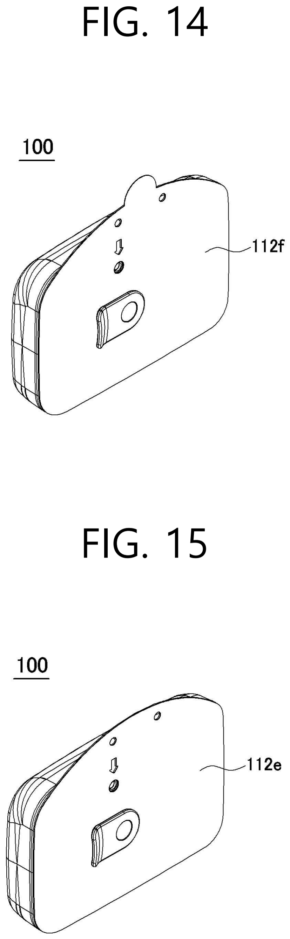

Further, referring to **FIG. 14** and **FIG. 15****,** the contact surface 112 of the housing 110 includes an adhesive sheet 112e that allows attachment to the injection target. The adhesive sheet 112e is formed larger than the contact surface 112 and extends around a periphery of the contact surface 112. A release paper 112f is attached to the adhesive sheet 112e. **FIG. 14** illustrates a state in which the release paper 112f is attached. When the release paper 112f is removed, the adhesive sheet 112e is exposed as shown in **FIG. 15****.** Further, a pre-formed incision portion is provided at a central portion of the release paper 112f. Thus, the release paper 112f can be easily removed before the device is attached to the injection target.

Unlike conventional drug injection devices, the patch-type drug injection device 100 of the present disclosure is manufactured to have a large area and a small thickness, thereby enhancing the stability of attachment to the user.

It would be understood by a person with ordinary skill in the art that various changes and modifications may be made based on the above description without changing technical conception and essential features of the present disclosure. Thus, it is clear that the above-described embodiments are illustrative in all aspects and do not limit the present disclosure. The scope of the present disclosure is defined by the following claims. It shall be understood that all modifications and embodiments conceived from the meaning and scope of the claims and their equivalents are included in the scope of the present disclosure.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A patch-type drug injection device, comprising:
a housing including a lid and a contact surface; and
components disposed in a mounting space between the lid and the contact surface,
wherein the components include:
a drug reservoir in which a drug to be injected is stored;
a pump assembly configured to be driven to transfer the drug in the drug reservoir toward an injection target;
a circuit unit equipped with electronic components necessary for driving and controlling the pump; and
an injection assembly equipped with an insertion tube to be inserted into the injection target, and
the injection assembly is disposed at a central region of the mounting space, and
the drug reservoir, the pump assembly, and the circuit unit are disposed to surround the injection assembly.

2. The patch-type drug injection device of Claim 1,
wherein the mounting space is divided into first to third regions which extend in a direction perpendicular to an insertion direction of the insertion tube and surround the central region, and
the drug reservoir, the pump assembly, and the circuit unit are disposed in the first to third regions, respectively.

3. The patch-type drug injection device of Claim 1, further comprising:
an adhesive sheet provided on the contact surface.

4. The patch-type drug injection device of Claim 3,
wherein the adhesive sheet is formed larger than the contact surface and extends around a periphery of the contact surface.

5. The patch-type drug injection device of Claim 3, further comprising:
a release paper configured to protect the adhesive sheet,
wherein the release paper includes a pre-formed incision portion at its central portion.

6. The patch-type drug injection device of Claim 1,
wherein the housing further includes:
a case frame disposed in the mounting space and configured to fix the components.

7. The patch-type drug injection device of Claim 6,
wherein the case frame includes:
a guide portion extending along an insertion direction of the insertion tube inserted into a central portion of the case frame and configured to guide a movement of the insertion tube.

8. The patch-type drug injection device of Claim 7,
wherein the guide portion is formed into a cylindrical shape.

9. The patch-type drug injection device of Claim 1,
wherein the lid has a shooting hole formed in a region corresponding to the insertion tube.

10. The patch-type drug injection device of Claim 1,
wherein the lid has rounded corners.

11. The patch-type drug injection device of Claim 1,
wherein the pump assembly sucks a drug from the drug reservoir and delivers the drug toward the injection assembly.

12. The patch-type drug injection device of Claim 11,
wherein the pump assembly includes:
an electroosmotic pump that is an electrochemically driven; and
a port connector that provides an inflow path through which the drug flows from the drug reservoir to the electroosmotic pump and a discharge path through which the drug is discharged from the electroosmotic pump to the injection assembly.

13. The patch-type drug injection device of Claim 1,
wherein the drug reservoir is provided as a pouch-type bag formed of a polymer material.

14. The patch-type drug injection device of Claim 13,
wherein the pouch-type bag is formed to be flat.

15. The patch-type drug injection device of Claim 13,
wherein a sealing portion is formed along a periphery of the pouch-type bag to restrict expansion toward the periphery.

16. The patch-type drug injection device of Claim 1,
wherein the insertion tube is a needle.

17. The patch-type drug injection device of Claim 1,
wherein the insertion tube includes:
a needle; and
a cannula into which the needle is inserted and which is disposed toward the injection target.

18. The patch-type drug injection device of Claim 1, further comprising:
a sound output device coupled to an inner side of the lid.

19. The patch-type drug injection device of Claim 1,
wherein the contact surface includes a drug injection hole for supplying a drug to be stored in the drug reservoir from the outside.

20. The patch-type drug injection device of Claim 1,
wherein the contact surface includes a discharge port to draw the insertion tube from the injection assembly to the outside of the contact surface.

21. The patch-type drug injection device of Claim 1,
wherein the contact surface includes an air vent hole to reduce a pressure difference between the inside and the outside of the housing.

22. The patch-type drug injection device of Claim 1,
wherein the circuit unit includes a sound output circuit capable of stopping an operation of the sound output device provided within the housing, and
the contact surface includes a buzzer stop hole to provide a passage allowing access to the sound output circuit from the outside.
